# EUROPEAN PATENT SPECIFICATION

(11) **EP 1 091 731 B1**
(45) Date of publication and mention of the grant of the patent: **21.09.2005**
(21) Application number: 99928066.2
(22) Date of filing: 30.06.1999
(51) Int. Cl.: A61K 9/26, A61K 9/24, A61K 31/557

(54) **ANTI-INFLAMMATORY PHARMACEUTICAL FORMULATIONS**
ENTZÜNDUNGSHEMMENDE PHARMAZEUTISCHE FORMULIERUNGEN
FORMULATIONS PHARMACEUTIQUES ANTI-INFLAMMATOIRES

(30) Priority: 01.07.1998 GB 9814215
(43) Date of publication of application: 18.04.2001
(73) Proprietor: NORTON HEALTHCARE LIMITED, London E16 2QJ (GB)
(72) Inventor: WOOLFE, Austin John, North Weald, Essex CM19 6DL (GB); McIntyre, Gordon, Bishops Stortford, Herts CM23 3PW (GB); Vadilal Sheth, Nitin, Goshen, NY 10924 (US)
(86) International application number: PCT/GB1999/001951
(87) International publication number: WO 2000/001368

(56) References cited:
- EP-A- 0 196 546
- WO-A-91/16895
- WO-A-98/20870
- US-A- 3 488 418

## Description

This invention relates to pharmaceutical formulations of anti-inflammatory drugs, particularly non-steroid and anti-inflammatory drugs (NSAIDs).

These NSAIDs are used for the treatment of inflammatory conditions such as osteoarthritis or rheumatoid arthritis.
A side effect of the oral administration of NSAIDs particularly with long term usage, is a liability to ulcerogenic effects. NSAID induced ulcers in the stomach are potentially dangerous because few or no symptoms may be detected until significant damage has been caused. Certain prostoglandins, for example misoprostol have been shown to reduce and even prevent such ulcers.

It has been found experimentally that it is necessary for the prostaglandin to be released before the NSAID so as to protect the stomach from the effects of the NSAID. It is therefore preferable that the NSAID is coated to delay release. The coating may be a standard hydroxypropyl methyl cellulose coat of a thickness sufficient to delay release in the stomach for a short period, an enteric coat to delay NSAID release until it reaches the intestine, or a delay release coating to allow drug release over a period of time to permit less frequent dosing.

In addition the coating may act as a barrier between the NSAID and the prostaglandin to prevent decomposition of the prostaglandin caused by instability in the pressure of the NSAID.

EP-527887B discloses a pharmaceutical composition comprising a core of an NSAID surrounded by a coating containing the prostaglandin. The core is preferably coated with a barrier of enteric coat before a mantle coat is added. No experimental details are given. It appears that this dosage form is made by press-coating, ie a tablet core containing the drug is made, and coated before being put in a second tableting operation to cover the coated core. Such a procedure requires use of specialised equipment which is not a normal pharmaceutical production tool and hence would need significant investment.

According to the present invention an oral pharmaceutical dosage form comprises a tablet containing a non-asteroidal anti inflammatory drug and misoprostol, wherein the non-asteroidal anti inflammatory drug is in the form of coated pellets.

The NSAID is preferably but not exclusively one of reasonably low weight per standard dose. That is an NSAID where the usual dose is 200 mg or below. Examples of such NSAIDs include indomethacin, piroxicam, meloxicam, flubiprofen, naproxan, ketoprofen, tenoxicam or similar molecules. Most preferably the drug is diclofenac sodium.

Enteric coated or delay release coated pellets have not been widely used because many workers have found damage or cracking to the enteric or delay release coating during the tablet compression stage. The present invention will work most effectively if the coating remains intact during compression.

It is possible to produce such pellets by conventional means although care is needed to ensure coat cracking does not occur. Techniques which can be used include coating the drug on a non-pariel core preferably composed of inert sugar or similar substance and then overcoating with the required coating before compression.

A preferred method is to form pellets by co-acervation or alternatively by precipitation of the pellets from solution as described by Zaruboru, Fell and Collett, (Int.J.Pharm, 1995, 125, 151-5).

In another preferred technique the pellets may be formed by spheronisation, rotogranulation or a similar technique. Preferably the pellets should be soft enough to deform slightly under compression to avoid cracking but not too soft so as to deform significantly which will also cause cracking or rupture of the coat. A suitable mixture of drug with a suitable amount of an excipient or several excipients can be found by simple, routine experiments. Suitable excipients include polyvinyl pyrolidone, sugars and cellulose derivatives particularly microcrystalline cellulose. A coating for the pellets may employ cellulose derivatives eg hydroxypropyl methyl cellulose, methacrylic acid and derivatives eg methyl methacrylates for example, Eudragrit® (Rhom Pharma), especially Eudragrit L or S. Other standard enteric coating materials for example phthalates, eg cellulose acetate phthalate or preferably hydroxypropylacetate phthalate or polyvinylacetate phthalate. Mixtures of these and other materials may be used to produce delay release coated beads. Normally coating will include plasticisers eg polyethylene glycol, triacilin or phthalate esters.

It has been found in practice that smaller pellets are better for use in accordance with this invention, preferably between 0.25 mm to 1.5 mm in diameter. Most preferably pellets between 0.8 mm to 1.2 mm diameter are employed. Pellets of this diameter show less tendency to crack under the compression forces.

The external compression material will include a prostaglandin, preferably misoprostol together with inert excipients. The prostaglandin may be used neat or it may be preferably diluted on an inert material. A preferred material is misoprostol diluted on hydroxypropyl methyl cellulose or polyvinyl pyrolidone. Other diluents may be used. The other materials which may be employed include inert fillers, binders, lubricants and colorants as used in normal pharmaceutical tablet making. An especially useful material for this invention is microcrystalline cellulose. The dosage of prostaglandin will be chosen to be suitable to prevent or reduce stomach ulceration caused by the NSAID. A suitable dose of misoprostol is between 100 - 200 micrograms per tablet but this may be increased or decreased depending on the NSAID used.

The coated pellets and prostaglandin mixture are then compressed on conventional tableting equipment. Tablets may have a break line or break lines to facilitate smaller doses. The tablet may be film or sugar coated if required.

Bilayer tablets may be employed. The non-steroidal anti inflammatory drug and excipients may be compressed into the lower half of the tablet and the misoprostol together with excipients superimposed and pressed onto it. A barrier layer may be provided between the two active ingredient-containing layers. The misoprostol containing layer may incorporate excipients to facilitate rapid dissolution of this active ingredient.

The invention is further described by means of example but not in any limitative sense.

### EXPERIMENT 1

The following ingredients were employed.

| | | |
|---|---|---|
| | Diclofenac Pellets Enteric Coated 40% | 123 mg |
| | Misoprostol Dispersion on HPMC (1:100) | 20 mg |
| 1) | Microcrystaline Cellulose (Avicel 102) | 33 mg |
| 2) | Sodium Stored Glycollate | 3 mg |
| 3) | Hydrogenated Cottonseed Oil | 1 mg |

The excipients 1 + 2 and misoprostol were sieved through a 250 µm screen. The diclofenac pellets were added and blended for 15 min in a cube blender. The lubricant 3) was added and the mixture was reblended for 5 min and compressed at 180 mg/tablet.

### EXPERIMENT 2

The mixture from Experiment 1 was blended with the equivalent of another 100 mg of microcrystalline cellulose and was then compressed at 280 mg/tablet.

### EXPERIMENT 3

The following ingredients were employed.

| | | |
|---|---|---|
| Mix 1 | Diclofenac Pellets Enteric Coated 40% | 123 mg |
| | Microcrystalline Cellulose | 133 mg |
| | Sodium Starch Glycollate | 3 mg |
| | Hydrogenated Cottonseed Oil | 1 mg |
| Mix 2 | Misoprostol dilution (1:100) | 20 mg |
| | Microcrystalline Cellulose | 196 mg |
| | Sodium Starch Glycollate | 3 mg |
| | Hydrogenated Cottonseed Oil | 1 mg |

Mixture 1 was prepared with sieved excipients and then compressed to form a layer, having a weight of 260 mg. Mixture 2 was prepared and compressed on top of the diclofenac layer to atop weight of 120 mg, ie total 360 mg.

The resulting bilayer tablets were overcoated with an HPMC taste masking coat. The bead diameter was 1.05 to 1.16 mm.

### Results.

Experiment 1 using USP baskets
Dissolution in acid 0.1 MHCL for .2 hrs
Less than 4% release.

Dissolution in pH 6.8 buffer
98-106% release after 1 hr

Scanning Electronic Microscopy showed no breakage of the enteric coating of the pellets after compression.

## Claims

1. A pharmaceutical dosage form comprising a tablet containing a non-steroidal anti inflammatory drug and misoprostol, wherein the non-steroidal anti inflammatory drug is in the form of coated pellets.

2. A dosage form as claimed in claim 1 containing a uniform mixture of coated non-steroidal anti inflammatory pellets and misoprostol.

3. A dosage form as claimed in claim 1 comprising a bilayer tablet containing coated non-steroidal anti inflammatory pellets in one layer and misoprostol in a second layer.

4. A dosage form as claimed in any preceding claim wherein the pellets include an overcoating of a barrier layer upon a pellet including a layer of non-steroidal anti inflammatory drugs sprayed or otherwise coated on a non-pariel core.

5. A dosage form as claimed in any preceding claim wherein the coating is an enteric coating.

6. A dosage form as claimed in claim 5 wherein the enteric coating is selected from: a methylmethacrylate copolymer, a polyvinylacetate phthalate, cellulose acetate phthalate, or hydroxypropylmethyl cellulose phthalate.

7. A dosage form as claimed in claim 6 wherein the enteric coat includes a plasticiser.

8. A dosage form as claimed in any of claims 5 to 7 including a barrier inert coat disposed between the drug core and the enteric coating.

9. A dosage form as claimed in claim 7 wherein the barrier coat is a cellulose derivative.

10. A dosage form as claimed in any of claims 1 to 4 wherein the pellets are coated with a barrier coat adapted to delay release of the non-steroidal anti inflammatory drug.

11. A dosage form as claimed in any of claims 1 to 4 wherein the pellets are coated with a delay release coat adapted to release the drug throughout the gastrointestinal tract.

12. A dosage form as claimed in claim 11 wherein the delay release coat is formed from a methacrylate polymer or a mixture of a methacrylate polymer and a cellulose derivative.

13. A dosage form as claimed in any preceding claim wherein the pellets have a diameter of 0.25 to 1.5 mm.

14. A dosage form as claimed in claim 13 wherein the pellets have a diameter of 0.8 to 1.2 mm.

15. A dosage form as claimed in any preceding claim including one or more excipients selected from binders, lubricants, colorants, bulking agents and disintegrants.

16. A dosage form as claimed in any preceding claim wherein the non-steroidal anti inflammatory drug is diclofenac.

17. A dosage form as claimed in any of claims 1 to 15 wherein the non-steroidal anti inflammatory drug is ketoprofen.

18. A dosage form as claimed in any of claims 1 to 15 wherein the non-steroidal anti inflammatory drug is naproxen.

19. A dosage form as claimed in any of claims 1 to 15 wherein the non-steroidal anti inflammatory drug is selected from piroxicam and meloxicam.

20. A dosage form as claimed in any preceding claim comprising a tablet overcoated with a sugar or cellulose film barrier coating.

21. A dosage form as claimed in any preceding claim comprising a tablet overcoated with a barrier or taste masking coating.

22. A dosage form as claimed in any preceding claim comprising a tablet wherein the ratio of diclofenac to excipients either in the whole tablet or in the diclofenac layer is between 70:30 and 30:70 parts by weight.

23. A method of manufacture of a pharmaceutical dosage form as claimed in claim 1 wherein the pellets are formed by extrusion and spheronisation of a mixture containing a non-steroidal anti inflammatory drug, followed by coating with a barrier coat.

24. A method of manufacture of a dosage form as claimed in claim 1 wherein the pellets are made by coasservation or precipitation from solution.

25. A method as claimed in claim 24 wherein an enteric coat is formed by contacting solutions of an alkali salt of a non-steroidal anti inflammatory drug, and enteric form forming polymer and an acid.

26. A method as claimed in claim 24 or 25 wherein the misoprostol is absorbed onto hydroxypropylmethylcellulose or other cellulose derivative prior to incorporation into a tablet.

27. A method of manufacture of a pharmaceutical tablet comprising the steps of mixing a coated pellet containing a non-steroidal anti inflammatory drug together with a powder containing misoprostol absorbed on a cellulose, polyvinylchloride or other excipient optionally together with one or more binding agents, bulking agents, disintegrants and lubricants and compressing the mixture to form tablets.

28. A method of manufacture of a pharmaceutical bilayer tablet consisting of mixing coated pellets containing a non-steroidal anti inflammatory drug with optional excipients selected from binders, bulking agents, disintegrants and lubricants; compressing the mixture to form the bottom half of a tablet and superimposing a mixture of misoprostol absorbed on a cellulose or polyvinylchloride or other material together with or more optional excipients selected from binders, bulking agents, disintegrants and lubricants; to form a tablet suitable for human administration.

## Patentansprüche

1. Pharmazeutische Dosierungsform, umfassend eine Tablette, die ein nicht-steroidales entzündungshemmendes Arzneimittel und Misoprostol enthält, wobei das nicht-steroidale entzündungshemmende Arzneimittel in Form beschichteter Pellets vorliegt.

2. Dosierungsform nach Anspruch 1, enthaltend eine einheitliche Mischung aus beschichteten nicht-steroidalen entzündungshemmenden Pellets und Misoprostol.

3. Dosierungsform nach Anspruch 1, umfassend eine Zweischicht-Tablette, enthaltend beschichtete nicht-steroidale entzündungshemmende Pellets in einer Schicht und Misoprostol in einer zweiten Schicht.

4. Dosierungsform nach irgendeinem vorangehenden Anspruch, wobei die Pellets einen Überzug aus einer Sperrschicht beinhalten, über einem Pellet; das eine Schicht aus nicht-steroidalen entzündungshemmenden Arzneimitteln gesprüht oder in sonstiger Weise auf einem non-pariel-Kern beinhaltet.

5. Dosierungsform nach irgendeinem vorangehenden Anspruch, wobei die Beschichtung eine enterische Beschichtung ist.

6. Dosierungsform nach Anspruch 5, wobei die enterische Beschichtung gewählt ist aus: einem Methylmethacrylat-Copolymer, einem Polyvinylacetatphthalat, Celluloseacetatphthalat oder Hydroxypropylmethylcellulosephthalat.

7. Dosierungsform nach Anspruch 6, wobei die enterische Beschichtung einen Weichmacher beinhaltet.

8. Dosierungsform nach irgendeinem der Ansprüche 5 bis 7, beinhaltend eine inerte Sperrschicht, die zwischen dem Arzneimittelkern und der enterischen Beschichtung angeordnet ist.

9. Dosierungsform nach Anspruch 7, wobei die Sperrschicht ein Cellulose-Derivat ist.

10. Dosierungsform nach irgendeinem der Ansprüche 1 bis 4, wobei die Pellets mit einer Sperrschicht beschichtet sind, die so ausgelegt ist, daß sie die Freisetzung des nicht-steroidalen entzündungshemmenden Arzneimittels verzögert.

11. Dosierungsform nach irgendeinem der Ansprüche 1 bis 4, wobei die Pellets mit einer die Freisetzung verzögernden Schicht beschichtet sind, die so ausgelegt ist, daß sie das Arzneimittel innerhalb des gesamten Gastrointestinal-Trakts freisetzt.

12. Dosierungsform nach Anspruch 11, wobei die Schicht mit verzögerter Freisetzung aus einem Methacrylatpolymer oder einer Mischung aus einem Methacrylatpolymer und einem Cellulose-Derivat gebildet ist.

13. Dosierungsform nach irgendeinem vorangehenden Anspruch, wobei die Pellets einen Durchmesser von 0.25 bis 1,5 mm aufweisen.

14. Dosierungsform nach Anspruch 13, wobei die Pellets einen Durchmesser von 0,8 bis 1,2 mm aufweisen.

15. Dosierungsform nach irgendeinem vorangehenden Anspruch, beinhaltend einen oder mehrere Exzipienten, gewählt aus Bindemitteln, Gleitmitteln, Färbemitteln, Volumenfüllmitteln und Desintegrationsmitteln.

16. Dosierungsform nach irgendeinem vorangehenden Anspruch, wobei das nicht-steroidale entzündungshemmende Arzneimittel Diclofenac ist.

17. Dosierungsform nach irgendeinem der Ansprüche 1 bis 15, wobei das nicht-steroidale entzündungshemmende Arzneimittel Ketoprofen ist.

18. Dosierungsform nach irgendeinem der Ansprüche 1 bis 15, wobei das nicht-steroidale entzündungshemmende Arzneimittel Naproxen ist.

19. Dosierungsform nach irgendeinem der Ansprüche 1 bis 15, wobei das nicht-steroidale entzündungshemmende Arzneimittel aus Piroxicam und Meloxicam gewählt ist.

20. Dosierungsform nach irgendeinem vorangehenden Anspruch, umfassend eine Tablette, die mit einer Zucker- oder Cellulosefilmsperrbeschichtung überzogen ist.

21. Dosierungsform nach irgendeinem vorangehenden Anspruch, umfassend eine Tablette, die mit einer Sperr- oder Geschmacksmaskierungsbeschichtung überzogen ist.

22. Dosierungsform nach irgendeinem vorangehenden Anspruch, umfassend eine Tablette, wobei das Verhältnis von Diclofenac zu Exzipienten entweder in der gesamten Tablette oder in der Diclofenacschicht zwischen 70:30 und 30:70 Gewichtsteilen beträgt.

23. Verfahren zur Herstellung einer pharmazeutischen Dosierungsform nach Anspruch 1, wobei die Pellets gebildet werden durch Extrusion und Sphäronisierung einer Mischung, die ein nicht-steroidales entzündungshemmendes Arzneimittel enthält, gefolgt von der Beschichtung mit einer Sperrschicht.

24. Verfahren zur Herstellung einer Dosierungsform nach Anspruch 1, wobei die Pellets durch Coazervation oder Fällung aus der Lösung hergestellt werden.

25. Verfahren nach Anspruch 24, wobei eine enterische Beschichtung gebildet wird durch Kontaktieren von Lösungen eines Alkalisalzes eines nicht-steroidalen entzündungshemmenden Arzneimittels und eines eine enterische Form bildenden Polymeren und einer Säure.

26. Verfahren nach Anspruch 24 oder 25, wobei das Misoprostol vor der Einbringung in eine Tablette auf Hydroxypropylmethylcellulose oder einem anderen Cellulose-Derivat absorbiert wird.

27. Verfahren zur Herstellung einer pharmazeutischen Tablette, umfassend die Schritte des Mischens eines beschichteten Pellets, enthaltend ein nicht-steroidales entzündungshemmendes Arzneimittel zusammen mit einem Pulver, enthaltend Misoprostol absorbiert auf einer Cellulose, Polyvinylchlorid oder einem anderen Exzipienten, wahlweise zusammen mit einem oder mehreren Bindemitteln, Volumenfüllmitteln, Desintegrationsmitteln und Gleitmitteln, und Verdichtens der Mischung zur Bildung von Tabletten.

28. Verfahren zur Herstellung einer pharmazeutischen Zweischicht-Tablette, bestehend aus dem Mischen beschichteter Pellets, enthaltend ein nicht-steroidales entzündungshemmendes Arzneimittel mit wahlweisen Exzipienten, gewählt aus Bindemitteln, Volumenfüllmitteln, Desintegrationsmitteln und Gleitmitteln; Verdichten der Mischung zur Bildung der Bodenhälfte einer Tablette und Darüberschichten einer Mischung aus Misoprostol absorbiert auf einer Cellulose oder Polyvinylchlorid oder einem anderen Material, zusammen mit einem oder mehreren wahlweisen Exzipienten, gewählt aus Bindemitteln, Volumenfüllmitteln, Desintegrationsmitteln und Gleitmitteln; um eine für die Verabreichung an Menschen geeignete Tablette zu bilden.

## Revendications

1. Forme posologique pharmaceutique comprenant un comprimé contenant une substance active constituée par un anti-inflammatoire non stéroïdien et du misoprostol, dans laquelle la substance active constituée par un anti-inflammatoire non stéroïdien est sous la forme de granules enrobés.

2. Forme posologique telle que revendiquée dans la revendication 1 contenant un mélange uniforme de granules enrobés d'anti-inflammatoire non stéroïdien et de misoprostol.

3. Forme posologique telle que revendiquée dans la revendication 1 comprenant un comprimé bicouche contenant des granules enrobés d'anti-inflammatoire non stéroïdien dans une couche et du misoprostol dans une deuxième couche.

4. Forme posologique telle que revendiquée dans l'une quelconque des revendications précédentes, dans laquelle les granules comprennent une couche de finition d'une couche barrière sur un granule comprenant une couche de substances actives constituées par des anti-inflammatoires non stéroïdiens pulvérisée ou déposée d'une autre manière sur un noyau non-pareil.

5. Forme posologique telle que revendiquée dans l'une quelconques des revendications précédentes, dans laquelle l'enrobage est un enrobage entérique.

6. Forme posologique telle que revendiquée dans la revendication 5, dans laquelle l'enrobage entérique est choisi parmi : un copolymère de méthacrylate de méthyle, un phtalate de poly(acétate de vinyle), un acétophtalate de cellulose ou un phtalate d'hydroxypropylméthylcellulose.

7. Forme posologique telle que revendiquée dans la revendication 6, dans laquelle l'enrobage entérique comprend un plastifiant.

8. Forme posologique telle que revendiquée dans l'une quelconque des revendications 5 à 7 comprenant un enrobage gastro-résistant inerte placé entre le noyau de substance active et l'enrobage entérique.

9. Forme posologique telle que revendiquée dans la revendication 7, dans laquelle l'enrobage gastro-résistant est un dérivé de cellulose.

10. Forme posologique telle que revendiquée dans l'une quelconque des revendications 1 à 4, dans laquelle les granules sont enrobés avec un enrobage gastro-résistant adapté pour libérer de manière retardée la substance active constituée par un agent anti-inflammatoire non stéroïdien.

11. Forme posologique telle que revendiquée dans l'une quelconque des revendications 1 à 4, dans laquelle les granules sont enrobés avec un enrobage à libération retardée adapté pour libérer la substance active dans la totalité du tractus gastro-intestinal.

12. Forme posologique telle que revendiquée dans la revendication 11, dans laquelle l'enrobage à libération retardée est formé d'un polymère méthacrylate ou d'un mélange d'un polymère méthacrylate et d'un dérivé de cellulose.

13. Forme posologique telle que revendiquée dans l'une quelconque des revendications précédentes, dans laquelle les granules présentent un diamètre de 0,25 à 1,5 mm.

14. Forme posologique telle que revendiquée dans la revendication 13, dans laquelle les granules présentent un diamètre de 0,8 à 1,2 mm.

15. Forme posologique telle que revendiquée dans l'une quelconque des revendications précédentes comprenant un ou plusieurs excipients choisis parmi les liants, les lubrifiants, les colorants, les agents diluants et les désintégrants.

16. Forme posologique telle que revendiquée dans l'une quelconque des revendications précédentes, dans laquelle la substance active constituée par un anti-inflammatoire non stéroïdien est le diclofénac.

17. Forme posologique telle que revendiquée dans l'une quelconque des revendications 1 à 15, dans laquelle la substance active constituée par un anti-inflammatoire non stéroïdien est le kétoprofène.

18. Forme posologique telle que revendiquée dans l'une quelconque des revendications 1 à 15, dans laquelle la substance active constituée par un anti-inflammatoire non stéroïdien est le naproxène.

19. Forme posologique telle que revendiquée dans l'une quelconque des revendications 1 à 15, dans laquelle la substance active constituée par un anti-inflammatoire non stéroïdien est choisie parmi le piroxicam et le méloxicam.

20. Forme posologique telle que revendiquée dans l'une quelconque des revendications précédentes, comprenant un comprimé enrobé avec un pelliculage barrière en sucre ou en cellulose.

21. Forme posologique telle que revendiquée dans l'une quelconque des revendications précédentes, comprenant un comprimé enrobé avec un enrobage barrière ou de masquage de goût.

22. Forme posologique telle que revendiquée dans l'une quelconque des revendications précédentes, comprenant un comprimé dans lequel le rapport entre le diclofénac et les excipients, soit dans la totalité du comprimé, soit dans la couche de diclofénac, est compris entre 70:30 et 30:70 parties en masse.

23. Procédé de fabrication d'une forme posologique pharmaceutique telle que revendiquée dans la revendication 1, dans lequel les granules sont formés par extrusion et sphéronisation d'un mélange contenant une substance active constituée par un anti-inflammatoire non stéroïdien, suivi par un enrobage avec un enrobage barrière.

24. Procédé de fabrication d'une forme posologique telle que revendiquée dans la revendication 1, dans lequel les granules sont obtenus par coacervation ou précipitation à partir d'une solution.

25. Procédé tel que revendiqué dans la revendication 24, dans lequel on forme un enrobage entérique par mise en contact de solutions d'un sel alcalin d'une substance active constituée par un anti-inflammatoire non stéroïdien et d'un polymère formant une forme entérique et d'un acide.

26. Procédé tel que revendiqué dans la revendication 24 ou 25, dans lequel le misoprostol est absorbé sur de l'hydroxypropylméthylcellulose ou un autre dérivé de cellulose avant son incorporation dans un comprimé.

27. Procédé de fabrication d'un comprimé pharmaceutique comprenant les étapes de mélange de granules enrobés contenant une substance active constituée par un anti-inflammatoire non stéroïdien avec une poudre contenant du misoprostol absorbé sur de la cellulose, du poly(chlorure de vinyle) ou un autre excipient éventuellement avec un ou plusieurs agents liants, agents diluants, désintégrants et lubrifiants et de compression du mélange pour former des comprimés.

28. Procédé de fabrication d'un comprimé pharmaceutique bicouche consistant à mélanger des granules enrobés contenant une substance active constituée par un anti-inflammatoire non stéroïdien avec des excipients éventuels choisis parmi les liants, les agents diluants, les désintégrants et les lubrifiants ; à comprimer le mélange pour former la moitié inférieure d'un comprimé et à superposer un mélange de misoprostol absorbé sur de la cellulose ou du poly(chlorure de vinyle) ou un autre matériau avec un ou plusieurs excipients éventuels choisis parmi les liants, les agents diluants, les désintégrants et les lubrifiants ; afin de former un comprimé approprié pour l'administration à des êtres humains.
